# EUROPEAN PATENT SPECIFICATION

(11) **EP 2 629 765 B1**
(45) Date of publication and mention of the grant of the patent: **06.07.2016**
(21) Application number: 11785110.5
(22) Date of filing: 20.10.2011
(51) Int. Cl.: A61K 31/122, A61P 17/02, A61P 31/12

(54) **METHODS AND COMPOSITIONS FOR TREATING POX VIRUS INFECTIONS WITH AN 1,4-NAPHTHOQUINONE AS ACTIVE AGENT**
VERFAHREN UND ZUBEREITUNGEN ZUR BEHANDLUNG VON INFEKTIONEN MIT POXVIREN MIT EINEM 1,4 NAPHTHOQUINON ALS WIRKSTOFF
MÉTHODE ET PRÉPARATION POUR LE TRAITEMENT DES INFECTION DE POXVIRUS AVEC UN AGENT ACTIF DE 1,4-NAPHTHOQUINONE

(30) Priority: 21.10.2010 WO PCT/IB2010/054766
(43) Date of publication of application: 28.08.2013
(73) Proprietor: Hügin, Ambros, 1206 Geneva (CH)
(72) Inventor: Hügin, Ambros, 1206 Geneva (CH)
(74) Representative: KATZAROV S.A.
(86) International application number: PCT/IB2011/054691
(87) International publication number: WO 2012/052956

(56) References cited:
- EP-A1- 1 595 536
- WO-A2-2007/141158
- CN-A- 101 342 170
- DE-A1-102004 034 744
- JP-A- 10 203 967
- US-A- 5 314 914
- US-A1- 2009 123 447

## Description

### FIELD OF THE INVENTION

The present invention relates to a method for treating a subject having viral induced lesions of the skin and/or mucous membranes with Naphthoquinone-containing pharmaceutical compounds.

### BACKGROUND OF THE INVENTION

Human viral infections from virus families such as *Poxviridae*, *Papillomaviridae*, and *Herpesviridae* can cause lesions of the skin and/or mucous membranes

Pox viruses are members of the *Poxviridae* family. The pox virus Molluscum contagiosum virus (MCV) is a member of the Sub-family Chordopoxvirinae, which also includes the Vaccinia virus. MCV is known to infect only humans. While no *in vitro* infection model exists, *in vivo* models use human skin transplanted immuno-deficient mice and are of limited use to study MCV infection.

Molluscum contagiosum (MC) is a benign infection of the skin and mucous membranes caused by MCV. Most people with MC have multiple lesions, which initiate as small papules that then enlarge to around 2-6 mm, but rarely exceed 1 cm in diameter. Most lesions resolve without scarring, but they may cause discomfort and/or itching. MCV is common worldwide and is responsible for about 1% of skin disorders in the USA, and this infection rate can rise to 2-8% in children. Although, when located around the eye, it can incite a toxic keratoconjunctivitis.

There are four closely related types of MCV, denoted MCV-1, MCV-2, MCV-3 and MCV-4. (Nakamura et al., 1995). MVC-1 is most common in children, while MCV-2 is more often seen in adults and is associated with sexual transmission. There is also a higher incidence of MCV infection in immuno-compromised patients, such as in the case of AIDS and patients undergoing chemotherapy.

Individual MCV lesions may go away on their own and generally last from 6-8 weeks to 2-3 months. However autoinoculation often occurs through scratching, and the disease may propagate extending the duration of the infection to from 8 months to 18 months (Tyring, 2003).

The use of menadione in a method for inhibiting replication of an RNA virus, namely HCV, is known from US 2009/123447. HCV, in contrast to MCV, is a single-stranded RNA virus that causes a systemic infection with extrahepatic manifestations, and requires a systemic therapy.

No single treatment option has been convincingly shown to be effective to date (van der Wouden et al., 2009). It should also be noted that some treatment options that can cause the skin on or near the lesions to rupture may also spread the infection further. Nonetheless, treatment may be sought for the following reasons: i) Medical reasons: bleeding, secondary infections, itching and discomfort, potential scarring, chronic keratoconjuctivitis or for ii) Social reasons: Cosmetic, embarrassment, fear of transmission to others, and social exclusion. Doctors recommend treating lesions in the genital area to prevent them from spreading (Tyring 2003).

Current treatments of MC include both surgical and medical options as follows:
Surgical Options include Cryosurgery, Evisceration, Curettage, Tape stripping and pulsed dye laser. Medical options include: Podophyllin and podofilox, Cantharidin, Iodine solution and salicylic acid plaster, Tretinoin, Cimetidine, Potassium hydroxide, Imiquimod Cidofovir, Adapelene, Trichloroacetic acid, a combination of Salicylic acid and lactic acid, Intralesional interferon alfa, topical injections of streptococcal antigen and OK-432.

The *Papillomaviridae* family contains the Human papillomavirus (HPV) member. Like all papillomaviruses, HPVs establish productive infections only in keratinocytes of the skin or mucous membranes. While the majority of the nearly 200 known types of HPV cause no symptoms in most people, some types can cause warts or verrucae, while others can, in a minority of cases, lead to cancers. Warts are infectious and can be autoinoculated and spread to other individuals by direct contact.

In very rare cases, HPV may cause epidermodysplasia verruciformis in immunocompromised individuals. There is currently no specific treatment for HPV infection.

There are various therapies for the treatment of warts, but none are considered truly effective as they typically fail to totally cure the lesions and do not prevent recurrence. A discussion of presently accepted therapies can be found in Gibbs (Gibbs et al. 2002; Gibbs et al. 2006). Recently, Imiquimod has been tested on warts with some limited therapeutic effect (Baker et al. 2011).

Genital HPV Infection - CDC Fact Sheet". Centers for Disease Control and Prevention (CDC). April 10, 2008. http://www.cdc.gov/std/HPV/STDFact-HPV.htm. Retrieved 13 November 2009 (verified October 17, 2011).

Mayo Clinic.com, Common warts, http://www.mayoclinic.com/print/common-warts/DS00370/DSECTION=all&METHOD=print (verified 17 October 2011).

HPV Vaccine Information For Young Women". Centers for Disease Control and Prevention (CDC). June 26, 2008. http://www.cdc.gov/std/hpv/STDFact-HPV-vaccine-young-women.htm. Retrieved 13 November 2009 (verified October 17, 2011).

American Cancer Society. "What Are the Risk Factors for Cervical Cancer?". Archived from the original on 19 February 2008.

http://web.archive.org/web/20080219151934/http://www.cancer.org/docroot/CRI/content/CRI_2_4_2 X_What_are_the_risk_factors_for_cervical_cancer_8.asp. Retrieved 21 February 2008 (verified October 17, 2011).

The *Herpesviridae* family is another well known virus family causing lesions of the skin and/or mucous membranes. Herpes simplex from the *Herpesviridae* family is a viral disease caused by both Herpes simplex virus type 1 (HSV-1) and type 2 (HSV-2). Infection with the herpes virus is categorized into one of several distinct disorders based on the site of infection. Common infection of the skin or mucosa may affect the face and mouth (orofacial herpes), genitalia (genital herpes), or hands (herpetic whitlow). More serious disorders occur when the virus infects and damages the eye (herpes keratitis), or invades the central nervous system, damaging the brain (herpes encephalitis). Oral herpes, the visible symptoms of which are colloquially called *cold sores* or *fever blisters,* infects the face and mouth. Oral herpes is the most common form of infection. Genital herpes, known simply as *herpes,* is the second most common form of herpes.

Herpes simplex is most easily transmitted by direct contact with a lesion or the body fluid of an infected individual. Transmission may also occur through skin-to-skin contact during periods of asymptomatic shedding.

Many cutaneous viral infections are caused by Herpes Simplex virus and, to date, there have been no cures or adequate treatments developed for viral infections, especially skin infections due to Herpes Simplex I and II (Wilhelmus 2010). Once infected, the virus remains in the body for life. Recurrent infections (outbreaks) may occur from time to time, especially in times of immune impairment such as HIV and cancer-related immune suppression.

For the moment no efficient topical treatment of the lesions of the skin and/or mucous membranes following viral infection through Molluscum contagiosum virus, Human papillomavirus or herpes simplex virus is available.

Thus, there remain significant unmet medical needs for efficient topical treatment of the skin and/or mucous membranes following these viral infections.

### SUMMARY OF THE INVENTION

The present invention provides a pharmaceutical composition comprising a therapeutically effective amount of a 1,4-naphthoquinone family compound for treating a subject having a viral induced lesion of the skin and/or mucous membrane caused by Molluscum contagiosum virus, wherein said 1,4-naphthoquinone family compound is selected from the group comprising
i) Menadione or a salt thereof, selected from the group comprising menadione bisulfite, menadione dimethylpyrimidol bisulphite, menadione sodium bisulfite, Menadione nicotinamide bisulfite, Menadione disphosphate tetrasodium salt, Menadione sodium phosphate, Menadione pyridinol bisulfite, menadione epoxide, and menadione sodium bisulfite trihydrate,
ii) Naphthoquinone or a salt thereof, selected from the group comprising Naphthoquinone bisulfite, Naphthoquinone dimethylpyrimidol bisulphite, Naphthoquinone sodium bisulfite, Naphthoquinone nicotinamide bisulfite, Naphthoquinone disphosphate tetrasodium salt, Naphthoquinone sodium phosphate, Naphthoquinone pyridinol bisulfite, Naphthoquinone epoxide, and Naphthoquinone sodium bisulfite trihydrate,
iii) Lawsone or a salt thereof selected from the group comprising Lawsone bisulfite, Lawsone dimethylpyrimidol bisulphite, Lawsone sodium bisulfite, Lawsone nicotinamide bisulfite, Lawsone disphosphate tetrasodium salt, Lawsone sodium phosphate, Lawsone pyridinol bisulfite, Lawsone epoxide, and Lawsone sodium bisulfite trihydrate
iv) Juglone or a salt thereof selected from the group comprising Juglone bisulfite, Juglone dimethylpyrimidol bisulphite, Juglone sodium bisulfite, Juglone nicotinamide bisulfite, Juglone disphosphate tetrasodium salt, Juglone sodium phosphate, Juglone pyridinol bisulfite, Juglone epoxide, and Juglone sodium bisulfite trihydrate
v) Plumbagin or a salt thereof selected from the group comprising Plumbagin bisulfite, Plumbagin dimethylpyrimidol bisulphite, Plumbagin sodium bisulfite, Plumbagin nicotinamide bisulfite, Plumbagin disphosphate tetrasodium salt, Plumbagin sodium phosphate, Plumbagin pyridinol bisulfite, Plumbagin epoxide, and Plumbagin sodium bisulfite trihydrate.

Further disclosed is a pharmaceutical composition that is administered before and/or concomitantly and/or after undergoing a second mechanical or chemical treatment.

### BRIEF DESCRIPTION OF THE DRAWINGS

**FIG. 1** shows the inhibitory effect of Menadione and Menadione bisulfite on Vaccinia virus replication. Values indicate average of 2 samples.
**FIG. 2** shows the inhibitory effect of various Naphthoquinone family compounds and selected other agents on Vaccinia virus. Abbreviations used: K1: Vitamin K1, K2: Vitamin K2, M: Menadione, MBS: Menadione bisulfite, BP: Benzoyl peroxide, CHP: Cumene hydroperoxide, HP: Hydrogen peroxide, t-BHP: Tert-butyl-hydroperoxide, DEM: Diethylmaleate, DNCB: Dinitrochlorobenzene, DNFB: Dinitrofluorobenzene, MSA: Mercaptosuccinic acid, D: Dicoumarol, and HQ: Hydroquinone.
**FIG. 3** represents the test of various agents (BP: Benzyl peroxide), at the indicated concentrations on triplicates of HaCaT cells in 96-well plates.
**FIGs. 4** A, B and C show the inhibitory effect of various concentrations of Menadione on Vaccinia virus replication in HeLa (Fig. 4A) and HaCaT cells (Fig. 4B). Inhibitory effect of various concentrations of Dicoumarol on Vaccinia virus replication in HeLa (Fig. 4C)
**FIG. 5** shows the direct virocidal effect of various Naphthoquinone family compounds and other various agents on Vaccinia virus (24 h exposure): Benzoyl peroxide, Dicoumarol, Dinitrochlorobenzene, Dinitrofluorobenzene, Vitamin K1, Vitamin K2, Menadione (M is fresh, M* is Menadione kept at RT for 2 months), Menadione bisulfite, and Control.

### DETAILED DESCRIPTION OF THE INVENTION

In the present invention, it has been unexpectedly found for the first time that a pharmaceutical composition comprising a 1,4-naphthoquinone family compound, a salt thereof, or an active metabolite of said compound can block the growth of poxviruses when applied to virus-induced lesions.

The 1,4-naphthoquinone family compounds are selected from the group comprising Menadione (2-methyl-1,4-naphthoquinone), Naphthoquinone (4a,8a-Dihydronapthalene-1.4-dione), Lawsone (2-hydroxy-1,4-naphthoquinone), Juglone ( 5-hydroxy-1,4-naphthalenedione) and Plumbagin (5-hydroxy-2-methyl-naphthoquinone), or a salt thereof.

An "active metabolite", as used herein, is a product produced through metabolism in the body of a specified compound, or salt thereof, and which exhibits the same biological activity as the specified compound.

Active metabolites of the 1,4-naphthoquinone family compound of the invention may be identified using routine techniques known in the art and their activities determined using tests. Such metabolites may result for example from the oxidation, reduction, hydrolysis, amidation, deamidation, esterification, deesterification, enzymatic cleavage, and the like, of the administered compound of the invention. Accordingly, the invention includes active metabolites of a compound of the invention, including compounds produced by a process comprising contacting a compound of this invention with a mammal for a period of time sufficient to yield a metabolic product thereof. Such metabolite may also be produced in vitro by oxidation, reduction, hydrolysis, amidation, deamidation, esterification, deesterification, or enzymatic cleavage of the corresponding compound, these compounds being selected from the group comprising Menadione (2-methyl-1,4-naphthoquinone), Naphthoquinone (4a,8a-Dihydronapthalene-1.4-dione), Lawsone (2-hydroxy-1,4-naphthoquinone), Juglone ( 5-hydroxy-1,4-naphthalenedione) and Plumbagin (5-hydroxy-2-methyl-naphthoquinone), or a salt thereof.

Throughout the specification, the term Menadione applies to and encompasses also its salts. The term Menadione salts includes all forms of this compound, including but not limited to Menadione bisulfite, Menadione dimethylpyrimidol bisulphite, Menadione sodium bisulfite, and Menadione sodium bisulfite trihydrate.

Throughout the specification, the term Naphthoquinone applies to and encompasses also its salts. The term Naphthoquinone salts includes all forms of this compound, including but not limited to Naphthoquinone bisulfite, Naphthoquinone dimethylpyrimidol bisulphite, Naphthoquinone sodium bisulfite, Naphthoquinone nicotinamide bisulfite, Naphthoquinone disphosphate tetrasodium salt, Naphthoquinone sodium phosphate, Naphthoquinone pyridinol bisulfite, Naphthoquinone epoxide, and Naphthoquinone sodium bisulfite trihydrate.

Throughout the specification, the term Lawsone applies to and encompasses also its salts. The term Lawsone salts includes all forms of this compound, including but not limited to Lawsone bisulfite, Lawsone dimethylpyrimidol bisulphite, Lawsone sodium bisulfite, Lawsone nicotinamide bisulfite, Lawsone disphosphate tetrasodium salt, Lawsone sodium phosphate, Lawsone pyridinol bisulfite, Lawsone epoxide, and Lawsone sodium bisulfite trihydrate.

Throughout the specification, the term Juglone applies to and encompasses also salts. The term Juglone salts includes all forms of this compound, including but not limited to Juglone bisulfite, Juglone dimethylpyrimidol bisulphite, Juglone sodium bisulfite, Juglone nicotinamide bisulfite, Juglone disphosphate tetrasodium salt, Juglone sodium phosphate, Juglone pyridinol bisulfite, Juglone epoxide, and Juglone sodium bisulfite trihydrate.

Throughout the specification, the term Plumbagin applies to and encompasses also its salts. The term Plumbagin salts includes all forms of this compound, including but not limited to Plumbagin bisulfite, Plumbagin dimethylpyrimidol bisulphite, Plumbagin sodium bisulfite, Plumbagin nicotinamide bisulfite, Plumbagin disphosphate tetrasodium salt, Plumbagin sodium phosphate, Plumbagin pyridinol bisulfite, Plumbagin epoxide, and Plumbagin sodium bisulfite trihydrate.

For example, it has been found for the first time that Menadione or a salt thereof can block the growth of poxviruses when applied to virus-induced lesions.

The prior art teaches various primary and secondary roles of Menadione as an experimental chemotherapeutic agent, and further teaches its use as an inexpensive micronutrient for livestock in many countries. Forms of Menadione are also added to some pet foods as a source of vitamin K. Menadione has been employed for the prevention and treatment of skin rash, secondary to anti EGFR therapy, as taught by WO2006113479 and WO2009114745.

The use of a1,4-naphthoquinone family compounds and in particular Menadione as a treatment for MCV had never been suggested nor practiced hitherto.

As used herein, the phrase "therapeutically effective amount" denotes an amount of a 1,4-naphthoquinone family compound sufficient to reduce and eventually eliminate the virus-induced lesions, and is less than the amount that can cause serious side effects through toxicity, for example. The therapeutically effective amount is that required to provide a benefit to risk ratio within the scope of sound judgement of someone skilled in the art.

As used herein, the phrase "subject having a viral induced lesion of the skin and/or mucous membranes" denotes a patient that has MCV-induced lesions or HPV-induced lesions or HSV-induced lesions of the skin and/or mucosa. In particular, MCV-induced skin lesions have the character of dome-shaped papules that show a central umbilication, are 2-6 mm in diameter and rarely exceed 1 cm in diameter. They may be present in groups, or be widely disseminated. Lesions may be located anywhere on the body, with a predilection for the face, trunk and extremities in children, and for the groin and genitalia in adults. Preferentially, the subject is animal. Most preferentially, the subject is a human.

As used herein, the phrase "pharmaceutically acceptable carrier" denotes any kind of non-active compound or formulation (e.g. creams, lotion or oils), which facilitate processing of the active ingredient (here 1,4-naphthoquinone family compounds such as but not limited to Menadione) into formulations that can be used pharmaceutically. For topical applications suitable penetrants may be used in the formulation. Such penetrants are generally known in the art.

In embodiments involving the treatment of MC, no limitation is placed on the genetic variants of MCV associated with the lesions. MCV, a member of the poxvirus family, is one of the largest viruses, only slightly smaller than the smallest bacteria. It has a complex DNA genome that replicates in the cytoplasm of infected cells. It cannot be grown in tissue culture or in eggs, and for this reason, Vaccinia virus was employed as a comparable virus model in the current in vitro studies. MCV has been grown in human foreskin grafted onto athymic mice but not in other laboratory animals. Humans are hosts for various types of pox viruses: Orthopoxvirus; which resembles variola (smallpox) and vaccinia, and which are ovoid, parapoxvirus; which are orf and mikers nodule viruses, which are cylindrical in shape, and an unclassified virus, with features that are intermediary between the two previous ones. These are intermediate in structure. They include MCV and Tanapox. In 1996, the primary structure and coding capacity of MCV was determined (Senkevich et al., 1996), and includes a coding region for a protein, that may affect the oxidative status of infected cell (Shisler, 1998). Analysis of the MCV genome has revealed that it encodes approximately 182 proteins, many of which have direct counterparts in orthopoxviruses. Restriction endonuclease analysis of the genomes by Nakamura et al. has identified 4 types (Nakamura et al., 1995). MCV 1 and MCV 11 have genomes of 185 kilobases and 195 kilobases respectively. MCV 111 and IV are very rare. No relationship between virus type and lesional morphology or anatomical distribution is known.

The expression "pharmaceutically acceptable" is employed herein in reference to compounds, materials, compositions in dosage forms that are within the scope of sound medical judgment, and suitable for use in contact with human skin, without causing excessive irritation, toxicity, allergic response or other problem or complication, that is commensurate with an acceptable benefit/risk ratio. Pharmaceutically acceptable includes all compounds that are dermatologically acceptable, such as creams, oils, lotions gels and the like containing 1,4-naphthoquinone family compound and/or any of its analogues.

Generally, the 1,4-naphthoquinone family compound active ingredient, for example Menadione, or a salt thereof, is formulated with one or more pharmaceutically acceptable carriers. Some non-limiting examples of such pharmaceutically acceptable carriers include: sugars and starches, gelatine, excipients such as cocoa butter, oils, glycols, and glycerol.

Generally, the 1,4-naphthoquinone family compound (for example Menadione, or a salt thereof) in compositions described herein can be formulated for topical applications using pharmaceuticals acceptable carriers well known in the cosmetic and pharmaceutical arts.

In preferred aspects, the formulations of the invention comprising one or more of the above active ingredients, non-active ingredients, emulsifiers and biological additives, should be of a form and consistency such that the active ingredients of the 1,4-naphthoquinone family compound (for example Menadione, or a salt thereof) or any of its analogues are well-preserved and stable over time, i.e. the active ingredients remain stable from the point of manufacture (or mixing by user) to the point of use. In further preferred aspects, it is desirable that the active ingredients of the 1,4-naphthoquinone family compound (for example Menadione, or a salt thereof) or any of its analogues remain substantially homogenously dispersed, or dissolved in the particular formulation used such that it is possible to deliver or apply consistent amounts of active ingredients as a function of the amount or the volume of the formulation applied.

In one embodiment of this invention, the 1,4-naphthoquinone family compound is formulated as a dermatologically-acceptable format such as, but not limited to, Ointments, Liniments, Pastes, Films, Hydrogels and other Gels, Liposomes, Transfersome vesicals, Creams, Lotions, balms, Medicated shampoos, Dermal patches, Transdermal patches, Transdermal sprays, Jet injector or the like, suitable for topical application.

Various skin creams can be employed in this invention. A cream is a topical preparation, usually used for application to the skin. Creams may be considered pharmaceutical products as even cosmetic creams are based on techniques developed by pharmacy and un-medicated creams are highly used in a variety of skin conditions (dermatomes). The use of the Finger tip unit concept may be helpful in guiding how much topical cream is required to cover different areas. Creams are semi-solid emulsions, and are mixtures of oil and water. They are divided into two types: oil-in-water (O/W) creams which are composed of small droplets of oil dispersed in a continuous aqueous phase, and water-in-oil (W/O) creams which are composed of small droplets of water dispersed in a continuous oily phase. Oil-in-water creams are more comfortable and cosmetically acceptable as they are less greasy and more easily washed off using water. Water-in-oil creams are more difficult to handle but many drugs, which are incorporated into creams, are hydrophobic and will be released more readily from a water-in-oil cream than an oil-in-water cream. Water-in-oil creams are also more moisturizing as they provide an oily barrier that reduces water loss from the stratum corneum, the outmost layer of the skin.

Creams are semisolid dosage forms containing one or more drug substances dissolved or dispersed in a suitable base. This term has traditionally been applied to semisolids that possess a relatively fluid consistency formulated as either water-in-oil (e.g., Cold Cream) or oil-in-water (e.g., Fluocinolone Acetonide Cream) emulsions. However, more recently the term has been restricted to products consisting of oil-in-water emulsions or aqueous microcrystalline dispersions of long-chain fatty acids or alcohols that are water washable and more cosmetically and aesthetically acceptable.

In embodiments of this invention, general concentrations of between 0.01 and 5% by weight of the pharmaceutical product are employed, and are sufficient for the treatment of virus-induced skin lesions.

In particular, treatment consists of painting the lesions with a cotton stick, applying a solution of 50mM of the 1,4-naphthoquinone family compound (such as Menadione) dissolved in DMSO to the tip of the lesion. After 4 weeks, the skin was normal and without scar or discoloration.

In the case of co-treatments, for instance with Dicoumarol, concentrations of 0.1% to 10% by weight are to be considered adequate. In the case of co-treatments, for instance with Vitamin C, concentrations of 0.1 - 5 % by weight, preferably in concentrations from 0.5 - 3 % by weight, most preferably from 1 to 2%, are considered.

The present invention also contemplates carrying out the method of a 1,4-naphthoquinone family compound of the invention (such as Menadione) administration with a second therapy aimed at treating the virus-induced lesion. In some aspects of the invention, the second therapy can be administered at the same time as the 1,4-naphthoquinone family compound treatment (such as Menadione); in other aspects it may be administered before or after the 1,4-naphthoquinone family compound treatment (such as Menadione).

In yet another aspect of this invention, the second treatment can be mechanical in origin, such as curettage, excision, surgery, cryotherapy, laser treatment, tape stripping etc. In other aspects, the second treatment can be of a chemical nature, such as trichloroacetic acid, salicylic acid.

In a further embodiment of this invention, a combination of 1,4-naphthoquinone family compound of the invention (such as Menadione), Dicoumarol and vitamin C may be advantageously employed in this invention. Additional compounds that can be advantageously employed in this invention include, but are not limited to compounds that can directly or indirectly interfere with mechanisms of cellular protection against oxidative stress. Such compounds include, but are not limited to agents such as dimethylmaleate, phorone, and buthionine sulfoxamine in concentrations of 0.1 - 5 % by weight, preferably in concentrations from 0.5 - 3 % by weight, most preferably from 1 to 2%.

### EXAMPLES

### Example 1 Clinical data

### Treatment of a patient:

An 11-year-old female patient, previously treated for Molluscum contagiosum infection with curettage, had two recurrences of Molluscum contagiosum. In each instance, a number of early lesions, which where in the elbow region or the lower jaw region respectively, were treated one to two times per day for 5 consecutive days. Treatment consisted of painting the lesions with a cotton stick, applying a solution of 50mM Menadione dissolved in DMSO to the tip of the lesion. After 4 weeks, the skin was normal and without scar or discoloration. Concentrations of Menadione, its analogues and additional compounds used in this invention are as indicated below:

In embodiments of this invention, in general concentrations of between 0.01 and 5% by weight of the pharmaceutical product are employed, and are sufficient for the treatment of virus-induced skin lesions. In the case of co-treatments, for instance with Dicoumarol, concentrations of 0.1% to 10% by weight are to be considered adequate. In the case of co-treatments, for instance with Vitamin C, in concentrations of 0.1 - 5 % by weight. In a further embodiment of this invention, a combination of Menadione, Dicoumarol and vitamin C may be advantageously employed in this invention. Additional compounds that can be advantageously employed in this invention include, but are not limited to compounds that can directly or indirectly interfere with mechanisms of cellular protection against oxidative stress. Such compounds include, but are not limited to agents such as dimethylmaleate, phorone, and buthionine sulfoxamine in concentrations of 0.1 - 5 % by weight.

### In vitro model

### Experimental protocols

Since MCV has not been shown to replicate in tissue culture, the related Vaccinia virus has been used in an *in vitro* model.

Experiments tested the effect of various compounds on Vaccinia virus infectivity.

Compounds were either preincubated with virus before infection of cell monolayers, or added to infected monolayers as described below. Virus plaque formation was the readout for theses experiments.

Preparation of Vaccinia virus strain WR, and description of a plaque assay to quantify virus is described in Earl et al., 2007.

Toxicity of test compounds on uninfected cell monolayers was measured using the standard tetrazolium dye MTT.

### Example 2

### Experimental Protocol

Inhibitory effect of Menadione and Menadione bisulfite on Vaccinia virus replication. Monolayers of HaCat and Hela cells in 96-well were infected with 1000 PFU (Plaque Forming Units) of Vaccinia virus WR.

After 1h, Menadione or Menadione bisulfite was added at the concentrations indicated.

After 1 day, the plates were submitted to 3 freeze-thaw cycles, the supernatant harvested and processed for titration of Vaccinia virus.

Values indicate average of 2 samples.

### Results

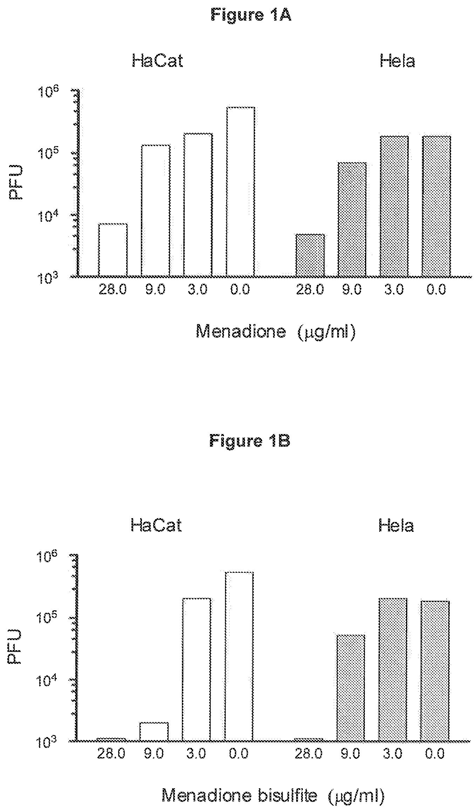

Fig.1 When tested on 2 established human keratinocyte cell lines, Menadione and Menadione bisulfite reduced virus progeny in a dose-dependend manner on both cell lines. At a concentration of 28 µm/ml, Vaccinia virus yields were always reduced by a factor > 10, as compared to controls.

### Example 3

### Experimental Protocol

Duplicates of 100 µl Vaccinia virus in wells of a 96-well plate were pre-incubated with 100 µl of 1 mM (final incubation concentration is 500 µM) of selected compounds.

After a 4 h incubation in said agents, Vaccinia virus was diluted 1000 fold and added to BS-C-1 monolayer cultures in 24-well plates to quantify virus plaque formation as a measure of remaining infectious virus particles.

Used compounds: K1: Vitamin K1, K2: Vitamin K2, M: Menadione, MBS : Menadione bisulfite, BP: Benzoyl peroxide, CHP: Cumene hydroperoxide, HP: Hydrogen peroxide, t-BHP: Tert-butyl-hydroperoxide, DEM: Diethylmaleate , DNCB: Dinitrochlorobenzene, DNFB: Dinitrofluorobenzene, MSA: Mercaptosuccinic acid, D: Dicoumarol, HQ: Hydroquinone.

### Results

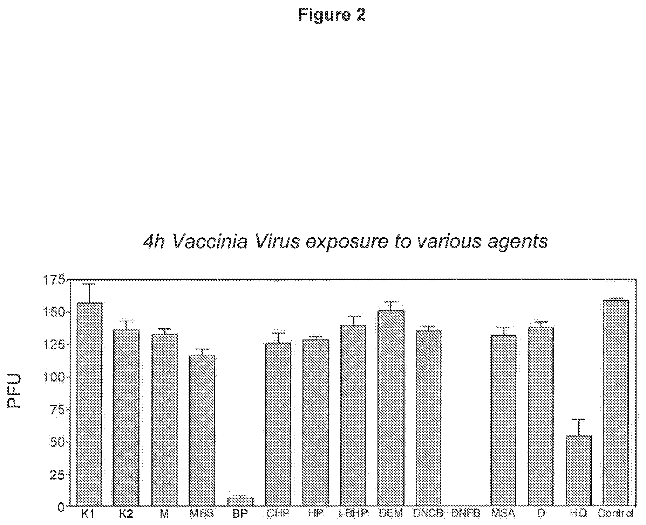

FIG. 2 Preincubation of Vaccinia virus with a series of coumpounds as indicated, variously reduced the infectivity of the virus, when quantified on subsequently infected monolayers of the BS-C-1 indicator cell line.

### Example 4

### Experimental Protocol

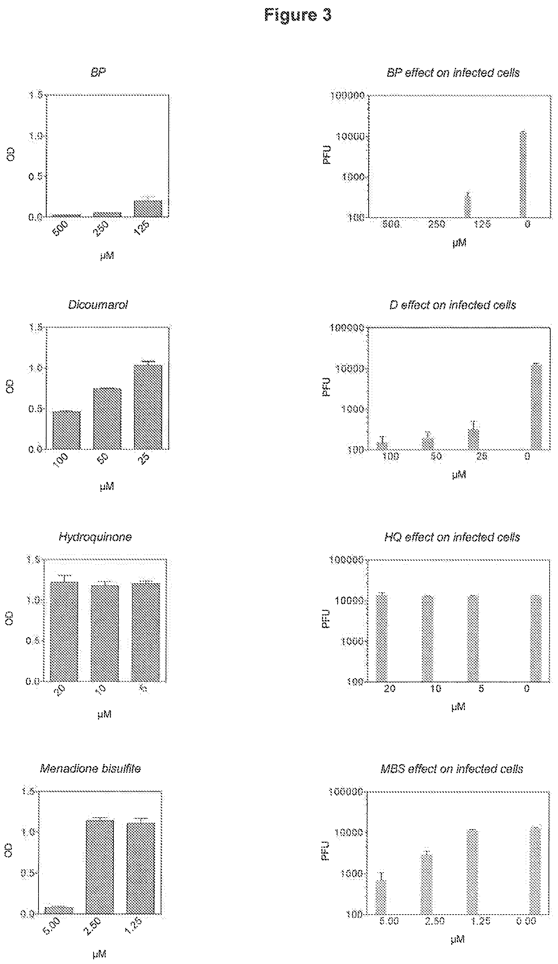

Test of various agents (BP: Benzoyl peroxide Figure 3 depicts), at the indicated concentrations on triplicates of HaCaT cells in 96-well plates (Fig.3).
- Toxicity on cells (left side of panel): Cell monolayers were exposed to agents as indicated, and 1 day later, cellular viability was analysed by a standard MTT assay (control OD was between 1.15 and 1.3).
- Inhibition of Vaccinia virus replication (right side of panel): HaCaT monolayers were infected with 1000 PFU of Vaccinia virus. After 2 h, agents as indicated were added. One day later, the plates were submitted to 3 freeze-thaw cycles and the wells harvested individually. Virus was then tittered according to standard virus titration procedures.

### Results

FIG 3 This Figure depicts the dose-dependend effect of indicated compounds, used at specific concentraions on two relevant parameters : i) virus inhibition (left panel) and ii) cell toxicity (right panel). While Hydroquinone at the shown concentrations had no effect on cell toxicity or virus replication, dose-dependent cell toxicity and inhibition of virus replication is shown by BP, Dicoumarol and Menadione bisulfite.

### Example 5

### Experimental Protocol

2x10⁴ Hela or 1x10⁴ HaCaT cells were seeded into 96-well plates and the plates incubated for 2 days. For toxicity studies, the cells were incubated in the presence of agent at the indicated concentration. After 1 day, a standard MTT assay was performed and optical density measured. For virus infection studies, triplicate cell monolayers were infected with 1000 PFU of Vaccinia virus in the presence of agent at the indicated concentrations, and added 2 h after virus was added to cells. Individual well harvested after 1 day. Virus was quantified in a standard plaque assay on BS-C-1 cells.

### Results

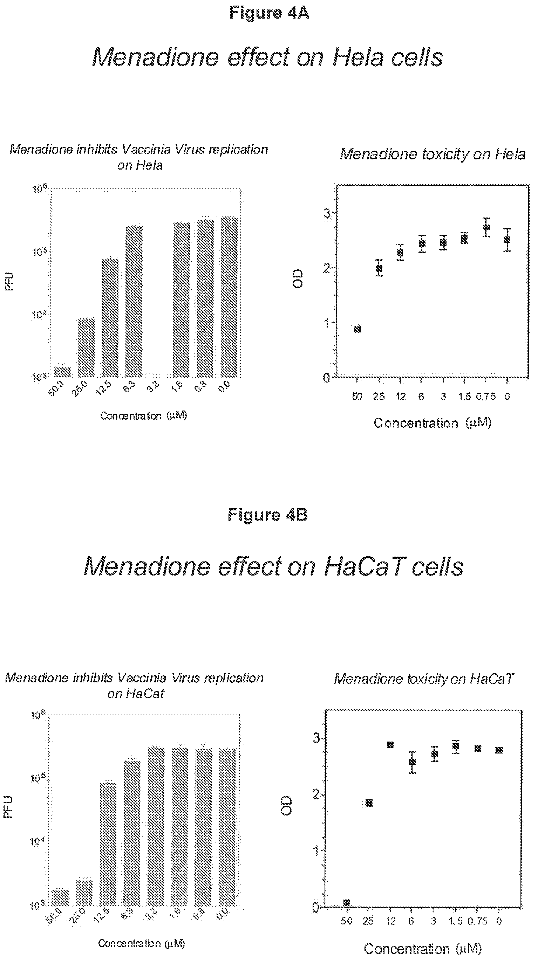

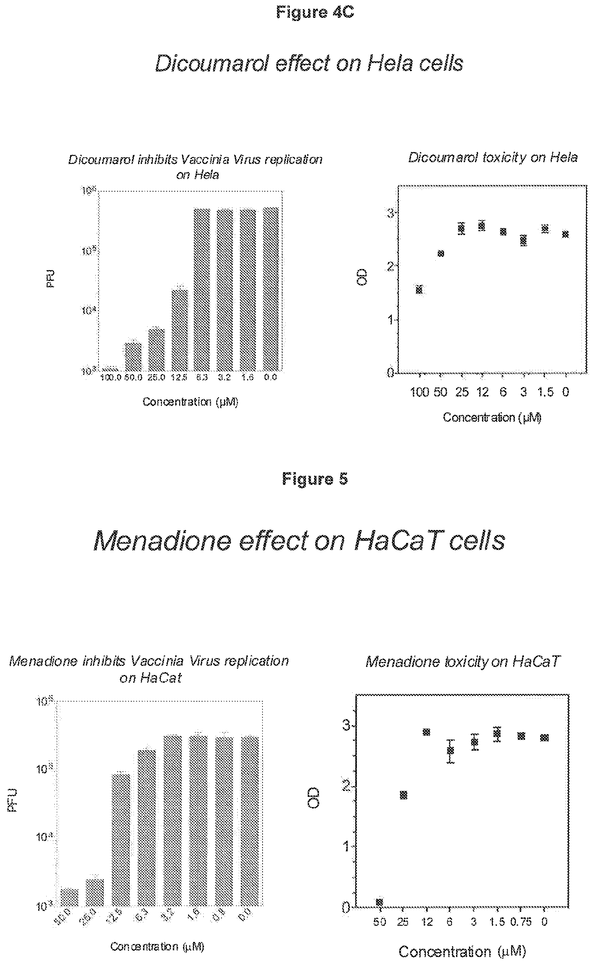

FIGs 4 A. B and C Here, Menadione is tested on 2 human keratinocyte cell lines at various concentrations for i) Vaccinia virus replication inhibition (left panel) and ii) cell toxicity (right panel). Similarly, Dicoumarol is tested on Hela cells. A dose-dependend effect is manifest in both test systems. A desirable effect would be i) a strong inhibition of virus replication and ii) a limited effect on cell viability. Indeed, in all three settings and at a concentration of 25 µM, test compounds show strong inhibition of virus replication (> 20x), and only limited effect on cell viability (< 30%).

### Example 6

### Experimental Protocol

### Direct virocidal effect of various agents on Vaccinia virus (24 h exposure)

Triplicates of Vaccinia virus in a 96-well plate were incubated with agents at 50 µM final concentration for 24 h at 37°C. After incubation, virus was diluted 1000-fold and quantified in a standard 24-well BS-C-1 plaque assay.

The various agents used were as follows: BP: Benzoyl peroxide, D: Dicoumarol, DNCB: Dinitrochlorobenzene, DNFB: Dinitrofluorobenzene, K1: Vitamin K1, K2:Vitamin K2, Menadione (M is fresh, M* is Menadione kept at RT for 2 months), MBS: Menadione bisulfite and Control.

### Results

FIG 5 Here, the direct virocidal effect of selected agents at a concentration of 50 µM on Vaccinia virus is quantitated in an infectivity assay. While most compounds tested have marginal effects on virus infectivity, Benzyl Peroxide reduced infectivity to almost half (43% reduction), and MBS inactivated 95% of infectious virus.

### REFERENCES

Baker DA, Ferris DG, Martens, MG, Fife KH, Tyring SK, Edwards L, Nelson A, Ault K, Trofatter KF, Liu T, Levy S, Wu J. 2011. Imiquimod 3.75% cream applied daily to treat anogenital warts: combined results from women in two randomized, placebo-controlled studies. Infect Dis Obstet Gynecol 2011:806105. Epub 2011 Aug 24.
Earl PL, Cooper N, Wyatt LS, Carroll MW. 2001. Preparation of cell cultures and vaccinia virus stocks. Curr protoc Mol Biol. Unit 16.16 Chapter 16: Unit 16.
Gibbs S, Harvey I, Sterling J, Stark R. 2002. Local treatments for cutaneous warts: systematic review. BJM 325: 461-468.
Gibbs S, Harvey I. 2006. Topical treatments for cutaneous warts. Cochrane Database Syst Rev Jul 19 ; (3) : CDC001781.
Nakamura J, Muraki Y, Yamada M, Hatano Y, Nii S. 1995. Analysis of molluscum contagiosum virus genomes isolated in Japan. J Med Virol. 46: 339-348.
Senkevich TG, Bugert JJ, Sisler JR, Koonin EV, Darai G, Moss B. 1996. Genome sequence of a human tumorigenic poxvirus: prediction of specific host response-evasion genes. Science 273: 813-816.
Shisler JL. Senkevich TG, Berry MJ, Moss B. 1998. Ultraviolet-induced cell death blocked by a selenoprotein from a human dermatotropic poxvirus. Science 279: 102-105.
Tyring SK. 2003. Molluscum contagiosum: the importance of early diagnosis and treatment. Am J Obstet Gynecol. 189 (3 Suppl): S12-16.
van der Wouden JC, van der Sande R, van Suijlekom-Smit LW, Berger M, Butler CC, Koning S. 2009. Interventions for cutaneopus molluscum contagiosum. Cochrane Database Syst Rev Oct 7: (4) : CD004767.
Wilhelmus KR. 2010. Antiviral treatment and other therapeutic interventions for herpes simplex virus in epithelial keratitis. Cochrane Database Syst Rev Dec 8: (12) : CD002898.

## Claims

1. A pharmaceutical composition comprising a therapeutically effective amount of a 1,4-naphthoquinone family compound for use in treating a viral induced lesion of the skin and/or mucous membrane caused by Molluscum contagiosum virus in a subject, wherein said 1,4-naphthoquinone family compound is selected from the group comprising
i) Menadione or a salt selected from the group comprising menadione bisulfite, menadione dimethylpyrimidol bisulphite, menadione sodium bisulfite, Menadione nicotinamide bisulfite, Menadione disphosphate tetrasodium salt, Menadione sodium phosphate, Menadione pyridinol bisulfite, menadione epoxide, and menadione sodium bisulfite trihydrate,
ii) Naphthoquinone or a salt selected from the group comprising Naphthoquinone bisulfite, Naphthoquinone dimethylpyrimidol bisulphite, Naphthoquinone sodium bisulfite, Naphthoquinone nicotinamide bisulfite, Naphthoquinone disphosphate tetrasodium salt, Naphthoquinone sodium phosphate, Naphthoquinone pyridinol bisulfite, Naphthoquinone epoxide, and Naphthoquinone sodium bisulfite trihydrate,
iii) Lawsone or a salt selected from the group comprising Lawsone bisulfite, Lawsone dimethylpyrimidol bisulphite, Lawsone sodium bisulfite, Lawsone nicotinamide bisulfite, Lawsone disphosphate tetrasodium salt, Lawsone sodium phosphate, Lawsone pyridinol bisulfite, Lawsone epoxide, and Lawsone sodium bisulfite trihydrate
iv) Juglone or a salt selected from the group comprising Juglone bisulfite, Juglone dimethylpyrimidol bisulphite, Juglone sodium bisulfite, Juglone nicotinamide bisulfite, Juglone disphosphate tetrasodium salt, Juglone sodium phosphate, Juglone pyridinol bisulfite, Juglone epoxide, and Juglone sodium bisulfite trihydrate
v) Plumbagin or a salt selected from the group comprising Plumbagin bisulfite, Plumbagin dimethylpyrimidol bisulphite, Plumbagin sodium bisulfite, Plumbagin nicotinamide bisulfite, Plumbagin disphosphate tetrasodium salt, Plumbagin sodium phosphate, Plumbagin pyridinol bisulfite, Plumbagin epoxide, and Plumbagin sodium bisulfite trihydrate.

2. The pharmaceutical composition for use according to claim 1, wherein said 1,4-naphthoquinone family compound, or said salt is administered topically.

3. The pharmaceutical composition for use according to any of the preceding claims, wherein the subject is a human.

4. The pharmaceutical composition for use according to claim 1, wherein said pharmaceutical composition is in the format of Ointments, Liniments, Pastes, Films, Hydrogels, Liposomes, Transfersome vesicals, Creams, Lotions, balms, Medicated shampoos, Dermal patches, Transdermal patches, Transdermal sprays, Jet injector or the like, suitable for topical application.

5. The pharmaceutical composition for use according to claim 1, comprising at least one additional pharmaceutically acceptable compound selected from the group comprising Dicoumarol, dimethylmaleate, phorone, buthionine sulfoxamine, or Vitamin C.

6. The pharmaceutical composition for use according to claim 1 wherein said pharmaceutical composition is administered, before and/or concomitantly and/or after undergoing a second mechanical or chemical treatment.

7. The pharmaceutical composition for use according to claim 6, wherein the second mechanical treatment is selected from the group of curettage, evisceration, excision, cryotherapy, laser treatment, or tape stripping.

8. The pharmaceutical composition for use according to claim 6, wherein the second chemical treatment is selected from the group of trichloroacetic acid or salicylic acid.

## Patentansprüche

1. Eine pharmazeutische Zusammensetzung, die eine therapeutisch wirksame Menge einer Verbindung der Familie 1,4-Naphthochinon zur Behandlung eines Patienten mit einer viral induzierten Läsion der Haut und/oder Schleimhaut, welche durch das *Molluscum contagiosum*-Virus verursacht wurde, worin die besagte Verbindung der Familie 1,4-Naphthochinon ausgewählt wird aus der Gruppe mit:
i) Menadion oder einem Salz, ausgewählt aus der Gruppe mit Menadionbisulfit, Menadion-Dimethylpyrimidol-Bisulfit, Menadionnatriumbisulfit, Menadion-Nicotinamid-Bisulfit, Menadion-Diphosphat-Tetranatriumsalz, Menadion-Natriumphosphat, Menadion-Pyridinol-Bisulfit, Menadion-Epoxid und Menadionnatriumbisulfit-Trihydrat,
ii) Naphthochinon oder einem Salz, ausgewählt aus der Gruppe mit Naphthochinonbisulfit, Naphthochinon-Dimethylpyrimidol-Bisulfit, Naphthochinonnatriumbisulfit, Naphthochinon-Nicotinamid-Bisulfit, Naphthochinon-Diphosphat-Tetranatriumsalz, Naphthochinon-Natriumphosphat, Naphthochinon-Pyridinol-Bisulfit, Naphthochinon-Epoxid und Naphthochinonnatriumbisulfit-Trihydrat,
iii) Lawson oder einem Salz, ausgewählt aus der Gruppe mit Lawsonbisulfit, Lawson-Dimethylpyrimidol-Bisulfit, Lawsonnatriumbisulfit, Lawson-Nicotinamid-Bisulfit, Lawson-Diphosphat-Tetranatriumsalz, Lawson-Natriumphosphat, Lawson-Pyridinol-Bisulfit, Lawson-Epoxid und Lawsonnatriumbisulfit-Trihydrat,
iv) Juglon oder einem Salz, ausgewählt aus der Gruppe mit Juglonbisulfit, Juglon-Dimethylpyrimidol-Bisulfit, Juglonnatriumbisulfit, Juglon-Nicotinamid-Bisulfit, Juglon-Diphosphat-Tetranatriumsalz, Juglon-Natriumphosphat, Juglon-Pyridinol-Bisulfit, Juglon-Epoxid und Juglonenatriumbisulfit-Trihydrat,
v) Plumbalgin oder einem Salz, ausgewählt aus der Gruppe mit Plumbalginbisulfit, Plumbalgin-Dimethylpyrimidol-Bisulfit, Plumbalginnatriumbisulfit, Plumbalgin-Nicotinamid-Bisulfit, Plumbalgin-Diphosphat-Tetranatriumsalz, Plumbalgin-Natriumphosphat, Plumbalgin-Pyridinol-Bisulfit, Plumbalgin-Epoxid und Plumbalginnatriumbisulfit-Trihydrat.

2. Die pharmazeutische Zusammensetzung zur Verwendung gemäss Anspruch 1, worin die besagte Verbindung der Familie 1,4-Naphthochinone oder das besagte Salz topisch verabreicht wird.

3. Die pharmazeutische Zusammensetzung zur Verwendung gemäss irgendeinem der vorhergehenden Ansprüche, worin das Subjekt ein Mensch ist.

4. Die pharmazeutische Zusammensetzung zur Verwendung gemäss Anspruch 1, worin die besagte pharmazeutische Zusammensetzung in der Form von Salben, Einreibemittel, Pasten, Folien, Hydrogelen, Liposomen, Transfersomevesikeln, Cremen, Lotionen, Balsam, medizinische Shampoos, dermale Pflaster, transdermale Pflaster, transdermale Sprays, Impfpistolen oder ähnliches, geeignet zur topischen Anwendung, besteht.

5. Die pharmazeutische Zusammensetzung zur Verwendung gemäss Anspruch 1, mit mindestens einer zusätzlichen pharmazeutisch annehmbaren Verbindung, ausgewählt aus der Gruppe mit Dicumarol, Maleinsäuredimethylester, Phoron, Buthioninsulfoximin, oder Vitamin C.

6. Die pharmazeutische Zusammensetzung zur Verwendung gemäss Anspruch 1, worin die besagte pharmazeutische Zusammensetzung vor und/oder gleichzeitig mit und/oder nach einer zweiten mechanischen oder chemischen Behandlung verabreicht wird.

7. Die pharmazeutische Zusammensetzung zur Verwendung gemäss Anspruch 6, worin die zweite mechanische Behandlung ausgewählt wird aus der Gruppe mit Kürettage, Eviszeration, Exzision, Kryotherapie, Laserbehandlung oder Tape-Stripping.

8. Die pharmazeutische Zusammensetzung zur Verwendung gemäss Anspruch 6, worin die zweite chemische Behandlung ausgewählt wird aus der Gruppe mit Trichloressigsäure oder Salizylsäure.

## Revendications

1. Une composition pharmaceutique comprenant une quantité thérapeutiquement efficace d'un composé de la famille de 1,4-naphthoquinone pour son utilisation dans le traitement d'une lésion de la peau et/ou de membrane muqueuse provoquée par le virus *Molluscum contagiosum* dans un sujet,
dans lequel ledit composé de 1,4-naphthoquinone est choisi dans le groupe comprenant
i) ménadione ou un sel sélectionné dans le groupe comprenant le bisulfite de ménadione, le bisulfite diméthyle-pyrimidol de ménadione, le bisulfite sodique de ménadione, le bisulfite de nicotinamide de ménadione, sel biphosphate tétrasodique de ménadione, le phosphate de sodium de ménadione, le bisulfite pyridinol de ménadione, l'époxyde de ménadione et le trihydrate de bisulfite sodique de ménadione,
ii) naphthoquinone ou un sel sélectionné dans le groupe comprenant le bisulfite de naphthoquinone, le bisulfite diméthyle-pyrimidol de naphthoquinone, le bisulfite sodique de naphthoquinone, le bisulfite de nicotinamide de naphthoquinone, sel biphosphate tétrasodique de naphthoquinone, le phosphate de sodium de naphthoquinone, le bisulfite pyridinol de naphthoquinone, l'époxyde de naphthoquinone et le trihydrate de bisulfite sodique de naphthoquinone,
iii) lawsone ou un sel sélectionné dans le groupe comprenant le bisulfite de lawsone, le bisulfite diméthyle-pyrimidol de lawsone, le bisulfite sodique de lawsone, le bisulfite de nicotinamide de lawsone, sel biphosphate tétrasodique de lawsone, le phosphate de sodium de lawsone, le bisulfite pyridinol de lawsone, l'époxyde de lawsone et le trihydrate de bisulfite sodique de lawsone,
iv) juglone ou un sel sélectionné dans le groupe comprenant le bisulfite de juglone, le bisulfite diméthyle-pyrimidol de juglone, le bisulfite sodique de juglone, le bisulfite de nicotinamide de juglone, sel biphosphate tétrasodique de juglone, le phosphate de sodium de juglone, le bisulfite pyridinol de juglone, l'époxyde de juglone et le trihydrate de bisulfite sodique de juglone,
v) plombagine ou un sel sélectionné dans le groupe comprenant le bisulfite de plombagine, le bisulfite diméthyle-pyrimidol de plombagine, le bisulfite sodique de plombagine, le bisulfite de nicotinamide de plombagine, sel biphosphate tétrasodique de plombagine, le phosphate de sodium de plombagine, le bisulfite pyridinol de plombagine, l'époxyde de plombagine et le trihydrate de bisulfite sodique de plombagine.

2. La composition pharmaceutique pour son utilisation selon la revendication 1, dans laquelle ledit composé de la famille de naphthoquinone ou ledit sel est administré par voie topique.

3. La composition pharmaceutique pour son utilisation selon l'une quelconque des revendications précédentes, dans laquelle le sujet est un être humain.

4. La composition pharmaceutique pour son utilisation selon la revendication 1, dans laquelle ladite composition pharmaceutique est en la forme de pommades, onguents, pâtes, films, hydrogels, liposomes, vésicules transfersomes, crèmes, lotions, baumes, shampoings médicamenteux, patchs dermiques, patchs transdermiques, sprays transdermiques, pistolet injecteur ou similaire, adaptés pour une application par voie topique.

5. La composition pharmaceutique pour son utilisation selon la revendication 1, comprenant au moins un composé pharmaceutiquement acceptable supplémentaire sélectionné dans le groupe comprenant Dicoumarol, diméthyle ester d'acide maléique, phorone, buthionine sulfoximine ou vitamine C.

6. La composition pharmaceutique pour son utilisation selon la revendication 1, dans laquelle ladite composition pharmaceutique est administrée, avant de suivre et/ou de façon concomitante et/ou après avoir suivi un deuxième traitement mécanique ou chimique.

7. La composition pharmaceutique pour son utilisation selon la revendication 6, dans laquelle le deuxième traitement mécanique est sélectionné dans le groupe de curetage, éviscération, excision, cryothérapie, traitement par laser ou Tape-Stripping.

8. La composition pharmaceutique pour son utilisation selon la revendication 6, dans laquelle le deuxième traitement chimique est sélectionné dans le groupe d'acide trichloro-acétique ou acide salicylique.
